# EUROPÄISCHE PATENTSCHRIFT

(11) **EP 1 931 705 B1**
(45) Veröffentlichungstag und Bekanntmachung des Hinweises auf die Patenterteilung: **07.09.2011**
(21) Anmeldenummer: 06792022.3
(22) Anmeldetag: 13.09.2006
(51) Int. Cl.: C07K 14/575, C07K 14/605, C12P 21/02, C12P 21/06

(54) **VERFAHREN ZUR AMIDIERUNG VON POLYPEPTIDEN MIT C-TERMINALEN BASISCHEN AMINOSÄUREN UNTER VERWENDUNG SPEZIFISCHER ENDOPROTEASEN**
METHOD FOR AMIDATING POLYPEPTIDES WITH BASIC AMINOACID C- TERMINALS BY MEANS OF SPECIFIC ENDOPROTEASES
PROCEDE POUR TRANSFORMER EN AMIDES DES POLYPEPTIDES AVEC DES AMINOACIDES BASIQUES C-TERMINAUX, AU MOYEN D'ENDOPROTEASES SPECIFIQUES

(30) Priorität: 27.09.2005 DE 102005046113
(43) Veröffentlichungstag der Anmeldung: 18.06.2008
(73) Patentinhaber: Sanofi-Aventis Deutschland GmbH, 65929 Frankfurt am Main (DE)
(72) Erfinder: RISSOM, Sebastian, 65926 Frankfurt am Main (DE); HABERMANN,Paul, 65926 Frankfurt am Main (DE); SALAGNAD, Christophe, 65926 Frankfurt am Main (DE); ZOCHER, Frank, 65926 Frankfurt am Main (DE); LANDRIC-BURTAIN, Laure, 75013 Paris (FR)
(86) Internationale Anmeldenummer: PCT/EP2006/008903
(87) Internationale Veröffentlichungsnummer: WO 2007/036299

(56) Entgegenhaltungen:
- EP-B1- 0 490 249
- WO-A-2004/035623
- WO-A-2006/015879
- WO-A-2006/058620
- THORKILDSEN CHRISTIAN ET AL: "Glucagon-like peptide 1 receptor agonist ZP10A increases insulin mRNA expression and prevents diabetic progression in db/db mice." JOURNAL OF PHARMACOLOGY AND EXPERIMENTAL THERAPEUTICS, Bd. 307, Nr. 2, November 2003 (2003-11), Seiten 490-496, XP002421605 ISSN: 0022-3565
- DAVEY M W ET AL: "TRYPSIN-MEDIATED SEMISYNTHESIS OF SALMON CALCITONIN" INTERNATIONAL JOURNAL OF PEPTIDE AND PROTEIN RESEARCH, MUNKSGAARD, COPENHAGEN, DK, Bd. 45, Nr. 4, 1. April 1995 (1995-04-01), Seiten 380-385, XP000493973 ISSN: 0367-8377
- BONGERS J ET AL: "SEMISYNTHESIS OF HUMAN GROWTH HORMONE-RELEASING FACTOR BY TRYPSIN CATALYZED COUPLING OF LEUCINE AMIDE TO A C-TERMINAL ACID PRECURSOR" INTERNATIONAL JOURNAL OF PEPTIDE AND PROTEIN RESEARCH, MUNKSGAARD, COPENHAGEN, DK, Bd. 40, Nr. 3/4, September 1992 (1992-09), Seiten 268-273, XP009037502 ISSN: 0367-8377
- SCHELLENBERGER V ET AL: "ATTEMPTS FOR QUANTIFYING THE S' SUBSITE SPECIFICITY OF SERINE PROTEASES" SCHOWEN, R. L. AND A. BARTH (ED.). ADVANCES IN THE BIOSCIENCES, VOL. 65. PEPTIDES AND PROTEASES: RECENT ADVANCES; SELECTED PAPERS PRESENTED AT THE 2ND INTERNATIONAL MEETING ON THE MOLECULAR AND CELLULAR REGULATION OF ENZYME ACTIVITY, HALLE, EAST GERM, 1987, Seiten 159-166, XP009079584 ISSN: 0-08-035726-1
- SCHELLENBERGER V ET AL: "PROTEASE-CATALYZED KINETICALLY CONTROLLED PEPTIDE SYNTHESIS" ANGEWANDTE CHEMIE. INTERNATIONAL EDITION, WILEY VCH VERLAG, WEINHEIM, DE, Bd. 30, Nr. 11, 1. November 1991 (1991-11-01), Seiten 1437-1449, XP000248151 ISSN: 1433-7851

## Beschreibung

Die vorliegende Erfindung betrifft ein Verfahren zur Herstellung C-terminal amidierter di- oder polybasischer Peptide, insbesondere solcher mit der biologischen Aktivität von GLP-1 oder deren Analoga oder Derivate.

Die Stabilität, biomedizinische Verfügbarkeit und Wirkdauer von pharmazeutisch relevanten Peptiden und Proteinen ist in hohem Maße von der Beschaffenheit des N-bzw. C-terminalen Molekülendes abhängig. Die Halbwertszeit von Biomolekülen wird durch C-terminale Verlängerung um basische Aminosäuren deutlich beeinflusst. Besonders gute pharmazeutische Wirksamkeiten kann man dann erreichen, wenn bei C-terminaler Verlängerung um mehr als eine basische Aminosäure die endständige C-terminale Aminosäure ein Aminosäureamid ist.

Die Herstellung solcher Wirkstoffe auf Peptidbasis kann, wenn die Peptide hinreichend klein sind, direkt über eine chemische Vollsynthese gemäß einem modifizierten Merrifield-Syntheseprotokoll erfolgen. Jedoch ergeben sich dann Limitierungen, wenn ein solches Peptid in großen Mengen benötigt wird. So müssen die in der Synthese einzusetzenden Aminosäuren zunächst hergestellt und gereinigt werden, um anschließend nach chemischer Modifikation in der Peptidsynthese als Reaktanden geeignet zu sein. Nach Entfernung der Schutzgruppen am Ende der Synthese kann das Zielpeptid bzw. Produkt dann gereinigt und als Pharmazeutikum formuliert werden. Je nach Zusammensetzung des Peptides kann es zudem bei der Synthese durch Nachbarschaftseffekte zu eingeschränkten Ausbeuten, Racemisierung oder zu Nebenproduktbildung durch Fehlkopplung bei einzelnen Kopplungsschritten kommen, wodurch die Gesamtausbeute oder die Reinheit des Produktes negativ beeinflusst werden kann. Für die Herstellung größerer Wertstoffmengen ist die Totalsynthese zu aufwendig. Die Herstellung über alternative, insbesondere biotechnische Verfahren wäre daher wünschenswert.

Seit langem sind Enzyme bekannt, die in der Lage sind, Peptide, C-terminal zu amidieren. Diese Enzyme tragen den Namen (Eipper et al. Mol. Endocrinol. 1987 Nov; 1 (11): 777) Peptidylglycine-alpha-amidating-enzyme (PAM). Die Herstellung und Reinigung solcher PAM-Enzyme ist dem Fachmann geläufig und detailliert beschrieben (M. Nogudi et al. Prot. Expr. Purif. 2003, 28: 293). Die Herstellung ist aber relativ zu der Herstellung anderer technischer Enzyme, wie z.B. Trypsin oder Carboxypeptidase, kostenintensiv.

Eine Alternative zu der ,in vitro'-Amidierung mittels PAM ergibt sich, wenn man das Enzym mit dem zu amidierenden Vorläuferprotein in derselben Wirtszelle coexprimiert. Dies erreicht man, indem man eine Gensequenz, die für eine PAM-Aktivität kodiert unter Kontrolle einer wirtsspezifischen Regulationssequenz in die Wirtszelle einbringt. Diese Expressionssequenz kann entweder stabil in die jeweilige chromosomale DNS-Sequenz eingebaut sein, oder auf einem zweiten Plasmid parallel zu dem Expressionsplasmid für das Zielprotein vorliegen, oder als zweite Expressionskassette auf demselben Vektor integriert sein, oder in einem polycistronischen Expressionsansatz in Phase mit der Gensequenz, die das Zielprotein kodiert unter Kontrolle der gleichen Promotorsequenz kloniert sein. Jedoch sind die beschriebenen Ausbeuten gering, so dass die Herstellung großer Mengen nur über entsprechende Fermentationsvolumina erreicht werden kann. Dies führt zu einem höheren kostenintensivem Reinigungsaufwand. Zudem erfolgt die Amidierung nicht quantitativ, so dass amidiertes von nicht amidiertem Wertprotein getrennt werden muss. Entscheidet man sich für einen sekretorischen Herstellungsprozess (z. B. Hong et al., Appl Biochem Biotechnol.2003;110, p.113-23) so ist zu bedenken, dass Proteine mit polybasischem C-Terminus nicht oder nur in sehr geringem Maße sekretiert werden.

Eine weitere Methode zur Amidierung basiert auf der Nutzung von proteinspezifischen Selbstspaltungsmechanismen (Cottingham et al. Nature Biotech. Vol.19, 974- 977, 2001). Jedoch ist die Steuerung dieser Reaktion nicht einfach und es kann zu einer Transthioesterifikation und damit zur Bildung unerwünschter Produkte kommen. Nachteilig in bezug auf die Ausbeute kann sich der relative große Fusionsproteinanteil auswirken.

Die oben beschriebenen Amidierungsverfahren gehen von einem um mindestens eine Aminosäure Glycin oder alternativ Inteinpeptid verlängerten C-Terminus des Zielpeptides aus. Peptide, die C-terminal Lysin tragen, lassen sich aber, wenn sie im weiteren Verlauf der Sequenz kein zusätzliches Lysin oder Arginin enthalten, als multimere Struktur herstellen, die man anschließend durch Verdau mit Trypsin oder Trypsin-ähnlichen Enzymen in den monomeren Baustein umwandeln kann. Dadurch lassen sich hohe Ausbeuten erzielen. Dies ist im Falle der oben beschriebenen Verfahren nicht möglich.

Die bekannten biotechnischen Herstellverfahren sind somit mit Nachteilen verbunden und die Aufgabe, Peptide oder Proteine, die C-terminal mehr als eine basische Aminosäure Lysin oder Arginin tragen und endständig amidiert sind, in großen Mengen kostengünstig herzustellen, kann noch nicht als befriedigend gelöst betrachtet werden.

Ein alternatives Herstellungsverfahren würde sich dann ergeben, wenn es gelänge eine um mindestens eine basische Aminosäure verkürzte Vorstufe des Zielpeptides in großen Mengen herzustellen und diese anschließend in einer enzymkatalysierten Semisynthese um Lysinamid bzw. Argininamid zu verlängern.

Levin et al. (Biochemical Journal 63:308-16;1956) beschreiben die Wirkung von Trypsin auf Lysinamid und verschiedene Poly-Lysinamid-Peptide. Die Ergebnisse der Autoren zeigen, dass Lysinamidderivate unter Trypsin-Einfluss nicht zu höhermolekularen Lysinamidderivaten umgesetzt werden, da die Hydrolyse der intermediär gebildeten Amide zur freien Säure im Vergleich zur Kupplungsreaktion schnell erfolgt. Daraus muss man schließen, dass Trypsin-katalysierte semisynthetische Verfahren, die die Herstellung von Peptiden mit C-ständigem poly-Lysinamid oder poly-Argininamid oder poly-Lys/Arg Mischsequenzen vorsehen, nicht, oder nur mit geringen Ausbeuten erfolgreich sind.

Es wurde nun ein peptidchemisches Verfahren gefunden, das überraschenderweise mit hohen Ausbeuten (d.h. >30%) die Trypsin-katalysierte Ligation von amidierten basischen Aminosäuren, deren Analoga oder Derivaten, an Peptide erlaubt, die C-terminal eine basische Aminosäure aufweisen.

Zudem konnte für das erfindungsgemäße Verfahren überraschenderweise beobachtet werden, dass die Einführung von Schutzgruppen (vgl. Pitraschke et al., Tetrahedron: Asymmetry 9, p1505-1518, 1998) wie z.B. -Boc (t-Butyloxycarbonyl), -Z (Benzyloxycarbonyl) oder -DDZ (Dimethylphenylpropyloxy-carbonyl) in den amidierten basischen Aminosäuren (Lysin-, Argininamid) nicht zu einer Verbesserung der Ligationsrektion in Bezug auf Effizienz und Selektivität führt. Das erfindungsgemäße Verfahren bietet somit den Vorteil, dass auf die Maskierung mit Schutzgruppen verzichtet werden kann, wodurch Ausbeuteverluste durch Entfernen der Schutzgruppe und die Entsorgung toxischer Reagenzien vermieden werden. Es ergeben sich hieraus enorme Kostenvorteile des Verfahrens gegenüber der chemischen Totalsynthese.

Thorkildsen et al. (The Journal of Pharmacology and Experimental Therapeutics, Vol. 307, No. 2, 490-496, 2003) beschreiben, dass der Agonist ZP10A des "Glucagon-Like Peptide 1" Rezeptors die Expression von Insulin mRNA verstärkt und die Progression von Diabetes in db/db Mäusen verhindert.

Im Europäischen Patent Nr. 0 490 249 wird ein Verfahren zur Herstellung von Growth Hormone Releasing Factor GRF(1-44)-NH₂ beschrieben, bei dem GRF (1-43)-OH mit Leu-NH₂ in der Gegenwart von Trypsin umgesetzt wird.

Schellenberger et al. (Advances in the Biosciences, Vol. 65, 159-166, 1987) beschreiben Studien zur Charakterisierung der Spezifität der S'-Untereinheit von Serinproteasen bei kinetisch kontrolierten Synthesen.

Ein Erfindungsgegenstand ist somit ein Verfahren zur Herstellung C-terminal amidierter di- oder polybasischer Peptide der allgemeinen Formel I

(AS)-X-NH₂ (I),

wobei
- AS: ein Peptid charakterisiert durch die Sequenz HGEGTFTSDL SKQMEEEAVR LFIEWLKNGG PSSGAPPSKK KKKK-NH₂ (SEQ ID No.1); oder HGEGTFTSDL SKQMEEEAVR LFIEWLKNGG PSSGAPPSKK KKKR-NH₂ (SEQ ID No. 4) und
- X: Lysin oder Arginin bedeutet;
worin ein Peptid charakterisiert durch die Sequenz
HGEGTFTSDL SKQMEEEAVR LFIEWLKNGG PSSGAPPSKK KKK (SEQ ID No. 7)
mit H-Arg-NH₂ oder H-Lys-NH₂ in Gegenwart eines Enzyms mit der biologischen Aktivität von Trypsin, wobei das Enzym die Fähigkeit hat, eine Peptidbindung C-terminal zu basischen Aminosäuren zu spalten, umgesetzt wird, und gegebenenfalls die erhaltene Verbindung der Formel I proteinchemisch aufgereinigt wird.

Weiterhin ist Gegenstand der Erfindung ein oben beschriebenes Verfahren, wobei im Falle der Herstellung des Peptids charakterisiert durch die Sequenz SEQ ID NO. 1 das molare Verhältnis des Peptids charakterisiert durch die Sequenz SEQ ID NO. 7 und H-Lys-NH₂ 1 : 44 und im Falle der Herstellung des Peptids charakterisiert durch die Sequenz SEQ ID NO. 4 das molare Verhältnis des Peptids charakterisiert durch die Sequenz SEQ ID NO. 7 und H-Arg-NH₂ 1 : 585 beträgt.

Weiterhin ist Gegenstand der Erfindung ein oben beschriebenes Verfahren, worin
a) ein Fusionspeptid exprimiert wird, das ein Peptid charakterisiert durch die Sequenz ID NO. 7 enthält;
b) das Peptid charakterisiert durch die Sequenz ID NO. 7 mittels enzymatischer Spaltung aus besagtem Fusionspeptid freigesetzt wird;
c) das Zwischenprodukt aus Schritt b), gegebenenfalls nach proteinchemischer Reinigung, in Gegenwart eines Enzyms mit der biologischen Aktivität von Trypsin mit H-Lys-NH₂ oder H-Arg-NH₂ umgesetzt wird; und
d) gegebenenfalls die erhaltene Verbindung an Peptide charakterisiert durch die Sequenzen SEQ ID NO. 1 oder 4 der Formel I proteinchemisch aufgereinigt und isoliert wird.

Weiterhin ist Gegenstand der Erfindung ein oben beschriebenes Verfahren, worin besagte Peptide der allgemeinen Formel I die biologische Aktivität von GLP-1, oder dessen Derivate oder Analoga aufweisen.

Weiterhin ist Gegenstand der Erfindung ein oben beschriebenes Verfahren, worin die Abspaltung von dem Fusionsprotein mittels Enterokinase, Faktor Xa, Genenase, Thrombin oder Trypsin geschieht.

Weiterhin ist Gegenstand der Erfindung ein oben beschriebenes Verfahren, worin das Fusionsprotein exprimiert wird in einem Expressionssystem ausgewählt aus der Gruppe enthaltend E.coli, S.carnosus, Salmonella, Bacillus subtilis, Pseudomonas flueorescens, K.lactis, P.pastoris, Schizosaccharomyces pombe und S. cerevisiae.

Im Sinn der vorliegenden Erfindung bedeuten:
Der Begriff "Analogon" einer Aminosäure bedeutet eine natürlich vorkommende Aminosäure, die nicht im genetischen Code codiert ist, sich aber zum Einbau in eine Peptidkette eignet. Beispiele für Analoga einer Aminosäure sind Ornithin und Citrullin, und auch alle weiteren nicht natürlich vorkommenden Aminosäuren, die in der Seitenkette jedwede basische Funktion tragen, wie z.B. 2,4- Diaminobutansäure; 3-Methyl-ornithin; 4-Methyl-ornithin, 5-Amino-leucin; 4-Amino-leucin; 3-Amino-leucin; 5-Amino-norteucin;4-Amino-norleucin;3-Amino-norleucin; 4-Amino-norvalin; 3-Amino-norvalin; 6-Methyl-lysin; 5-Methyl-lysin; 4-Methyl-lysin; 3-Methyl-lysin.

Der Begriff "Derivat" einer Aminosäure bedeutet eine Aminosäure oder ein Analogon einer Aminosäure, die durch eine oder mehrere chemische Gruppen substituiert ist. Beispiele für solche chemischen Gruppen sind in der Peptidchemie übliche Schutzgruppen, wie -Boc (t-Butyloxycarbonyl), -Z (Benzyloxycarbonyl), -DDZ (Dimethylphenylpropyloxy-carbonyl), -Fmoc (N-alpha-(9-fluorenylmethyloxycarbonyl), - 2-Bromo-Z, -2-Chloro-Z, -Tfa (Trifluoroacetyl), -Nicotinoyl, -4-Nitro-Z, 2-Picolinoyl, -Tos (4-Toluenesulphonyl), -For (Formyl), -Biotinyl, -Dansyl, -Dnp (Dinitrophenyl), -Mca (Monochloracetyl), -Mtt (N-Methyltrityl), -Nde (N-1-(4-nitro-1,3-dioxoindan-2-ylidene)ethyl), -Acetimidoyl, -Acetyl, - Myristoyl, -Palmitoyl, N-lithocholyl - γ - glutamyl oder - ω - carboxyheptadecanoyl.

Weiterhin ist eine solche Gruppe eine Gruppe C(O)-(C₆-C₂₄)Alkyl, eine Gruppe C(O)-(C₆-C₂₄)Alkenyl, eine Gruppe C(O)-(C₆-C₂₄)Alkandienyl, oder eine Gruppe C(O)-(C₆-C₂₄)Alkantrienyl, wobei sofern vorhanden Alkyl-, Alkenyl-, Alkandienyl- und Alkantrienylgruppen verzweigt oder geradkettig sein können. (C₁-C₆)Alkyl bedeutet ein Kohlenwasserstoffrest mit 1, 2, 3, 4, 5 oder 6 C-Atomen. Beispiele für (C₁-C₆)Alkylreste sind Methyl, Ethyl, n-Propyl, Isopropyl (1-Methylethyl), n-Butyl, Isobutyl (2-Methylpropyl), sec-Butyl (1-Methylpropyl), tert-Butyl (1,1-Dimethylethyl), n-Pentyl, Isopentyl, tert-Pentyl, Neopentyl, Hexyl. (C₆-C₂₄)Alkyl bedeutet entsprechend ein Kohlenwasserstoffrest mit 6 bis 24 C-Atomen. Alkylreste können geradkettig oder verzweigt sein. Als (C₆-C₂₄)Alkylreste sind Fettsäurereste bevorzugt, beispielsweise Hexyl, Octyl, Decanyl, Undecanyl, Dodecanyl, Tridecanyl, Tetradecanyl (Myristyl), Pentadecanyl, Hexadecanyl, Heptadecanyl, Octadecanyl (Stearyl), Nonadecanyl, Eicosanyl, Dicosanyl, 9,11-Dimethyl-tridecanyl, 11-Methyl-tridecanyl.

Weiterhin ist eine solche Gruppe eine Gruppe C(O)-Phenyl-(C₅-C₈)Heteroaryl-Phenyl, C(O)-Biphenyl oder C(O)-Terphenyl, wobei die Phenyl-, Biphenyl, Terphenyl- oder Heteroarylgruppen unsubstituiert sind oder substituiert sind mit einer oder zwei Gruppen ausgewählt aus der Gruppe (C₁-C₁₀)Alkyl oder O(C₁-C₁₀)Alkyl. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl.

Der Begriff "basische Aminosäure" bedeutet Lysin, Arginin, oder deren Derivate oder Analoga wie z.B. Ornithin oder Citrullin, vorzugsweise Lysin oder Arginin, insbesondere Lysin. Hierbei können Lysin oder Arginin anstelle der CarboxamidGruppe durch eine oder mehrere einer anderen reaktiven Gruppe derivatisiert sein, insbesondere ausgewählt aus der Gruppe von Aminosäureester, Peptidester, Anhydrid und Halogenid, und entsprechend in dem erfindungsgemäßen Verfahren eingesetzt werden.

Der Begriff "Enzym mit der biologischen Aktivität von Trypsin" bedeutet neben den bekannten und kommerziell verfügbaren Trypsinen aus den herkömmlichen Quellen wie Ratte, Rind, Schwein, Mensch, Hund, Maus deren Isoenzyme, Derivate oder Varianten auch Enzyme mit stark verwandten biochemischen Eigenschaften wie z. B. Cathepsin, Trypsin aus Fusarium oxysporum und aus Streptomyces (S. griseus, S. exfoliatus, S. erythraeus, S. fradiae und S. albidoflavus), Tryptase, Mastin, Acrosin, Kallikrein, Hepsin, Prostasin I, Lysylendopeptidase (Lys-C) und Endoproteinase-Arg-C (Clostripain).

Dabei ist dem Fachmann klar, dass diese Aufzählung nicht abschließend ist und im Rahmen der biotechnologischen Forschung stetig weitere Enzyme, Isoenzyme, Derivate oder Varianten entdeckt werden, die spezifisch C-terminal zu basischen Aminosäuren zu spalten vermögen. Alternativ lässt sich die Spezifität von Enzymen durch peptidchemische Modifikation oder Mutation auf DNS-Ebene verändern (Muteine). Zudem können die Spezifität und Aktivität von Enzymen durch geeignete Wahl der Reaktionsbedingungen deutlich modifiziert werden.

In der bevorzugten Ausführungsform des erfindungsgemäßen Verfahrens wird die Fähigkeit von Mikroorganismen ausgenutzt, heterologe Peptide herzustellen. Dazu übersetzt man die gewünschte Peptidsequenz in die entsprechende DNS-Sequenz, die an eine wirtsspezifische Promotorsequenz gekoppelt wird. Je nach Expressionsstrategie kann man dabei das Zielpeptid so exprimieren, dass es von den Zellen intrazellulär verbleibend direkt oder indirekt als Fusionspeptid gebildet wird.

Wählt man eine Fusionsstrategie unter Verwendung eines geeigneten Fusionspeptids, dann ist dem Fachmann klar, dass die Fusionspartner über ein Brückenglied miteinander verbunden sein müssen, das die spezifische Aufspaltung der Partner in einer Weise erlaubt, so dass der gewünschte N-Terminus des Zielpeptides nach der Prozessierung vorliegt. Im Sinne der vorliegenden Erfindung ist das Zielpeptid eine Verbindung der Formel II. Für die Ausgestaltung geeigneter Brückenglieder gibt es eine Vielzahl von Möglichkeiten, die dem Fachmann bekannt sind und die ständig erweitert werden. Wählt man z.B. die Aminosäure Methionin, so wird eine chemische Spaltung mit Halogencyan möglich. Wählt man z.B. als Brückenglied ein Pentapeptid der Sequenz DDDDK, so ist eine Spaltung mit Enterokinase möglich. Wählt man z.B. die Tetrapeptidsequenz IEGR, so kann die Spaltung über Faktor Xa erfolgen. Bei entsprechender Ausgestaltung kann man für Peptide, deren N-Terminus mit Histidin beginnen, Genenase^{®} als Prozessierungsenzym verwenden. Wird der N-Terminus durch das Dipeptid Gly-Ser charakterisiert, kann man als Brückenglied das Tetrapeptid LVPR wählen, so dass eine Erkennungs- und Spaltstelle für Thrombin entsteht. Obgleich ein Enzym mit der biologischen Aktivität von Trypsin erfindungsgemäß zur Peptidkupplung zum Einsatz kommt, ist bereits an dieser Stelle des erfindungsgemäßen Verfahrens der Einsatz eines solchen Enzyms grundsätzlich möglich. So kann zunächst Trypsin in wässrigem Milieu zur Abspaltung des Fusionsteils des Fusionspeptids benutzt werden, sofern Lysin oder Arginin als Brückenglied zwischen Fusionsteil und Zielpeptid eingefügt ist.

Alternativ kann man aber auch das Zielpeptid, wenn es exportkompatibel ist, in das Zellkulturmedium entweder in Form eines Fusionspeptids oder in seiner nativen Form ausschleusen. Dafür kann man rekombinante Wirtszellen, insbesondere solche von Mikroorganismen, vorzugsweise von Bakterien oder Hefen, verwenden. Wählt man Bakterienzellen als Expressionssystem hat man ferner die Option, direkt das Zielpeptid oder ein entsprechendes Fusionspeptid, welches das Zielpeptid, d.h. im Sinne der Erfindung eine Verbindung der Formel II, umfasst, in das Periplasma oder in das Kulturmedium auszuschleusen. Dabei ist dem Fachmann klar, dass der Export häufig dann eingeschränkt ist, wenn das C-terminale Ende aus mehr als einer basischen Aminosäure besteht.

Dem Fachmann sind die hierfür grundsätzlich zur Verfügung stehenden Wirtsorganismen und Methoden bekannt (vgl. z.B. Gellissen, Gerd (ed.) Production of Recombinant Proteins, ISBN 3-527-31036-3). Diese sind zum großen Teil auch kommerziell von einer Vielzahl von Anbietern erhältlich. Stellvertretend seien die Firmen New England Biolabs, Invitrogen und Roche genannt. In den Katalogbeschreibungen solcher Firmen finden sich Literaturzitate, die einen Überblick über die Technologie liefern. Dem Fachmann ist dabei auch klar, dass sich das Spektrum der zur Verwendung kommenden Mikroorganismen ständig erweitert, ebenso wie das biotechnologische Methodenrepertoire. Auch die in dieser Hinsicht spezielleren Ausführungsformen werden vom Gegenstand der vorliegenden Erfindung umfasst.

Stellvertretend werden beispielhaft folgende Wirts/Vektorsysteme genannt: Bakterien des Typs E.coli, S.carnosus, Salmonella, Bacillus subtilis oder Pseudomonas, insbesondere Pseudomonas fluorescens, sowie Hefen vom Typ K.lactis, P.pastoris, Schizosaccharomyces pombe und S. cerevisiae.

Dem Fachmann ist jedoch bewusst, dass diese beispielhaft genannten Systeme eine Vielzahl von Variationsmöglichkeiten bieten, die sich z.B. aus der Wahl geeigneter Promotoren oder anderer regulatorischer Nukleinsäuresequenzen, den genetischen Eigenschaften der Wirtszelle und der verwendeten Vektoren ergeben (z.B. bezüglich der Kopienzahl der DNS, der Wahl des Selektionsmittels etc.).

Dem Fachmann ist ebenfalls bewusst, dass für jedes Zielpeptid aufgrund seiner physikalisch chemischen Eigenschaften ein spezielles Verfahren zur Reinigung adaptiert werden muss, sofern dieses in isolierter Form bereitgestellt werden soll. Dies wird grundsätzlich durch die geeignete Kombination bekannter biochemischer bzw. biophysikalischer Trennmethoden erzielt. Dabei ist ebenfalls klar, dass ständig aufgrund neuartiger Materialien (z. B. für die Chromatographie) neue Möglichkeiten eröffnet werden, den gewünschten Reinigungserfolg zu erzielen bzw. zu optimieren.

Vorteilhaft im Sinne der vorliegenden Erfindung kann die Fusion des Vorläuferpeptids mit einer Peptidsequenz sein, die z.B. eine affinitätschromatographische Reinigung erlaubt.

Zur weiteren Ausgestaltung der Erfindung wurden Exendinderivate beispielhaft hergestellt, wie sie aus der US2004/0106547 bekannt sind. Solche von Exendin abgeleitete Peptide können aufgrund ihrer Blutzucker-senkenden Wirkung in der Behandlung von Diabetes oder anderen Stoffwechselstörungen, die z.B. zur Fettleibigkeit führen in der Medikamentenentwicklung eine wichtige Rolle spielen. Im pharmazeutischen Sinne ist es daher sinnvoll, solche Peptide in ausreichender Form zur Verfügung zu stellen.

Die internationale Patentanmeldung WO 02/066628 beschreibt ein Hirudinderivat, das C-terminal mit den Aminosäuren Lys -Arg endet. Das antithrombotische Wirkprofil lässt sich, dadurch verändern, dass man das C-terminale Arginin amidiert. Dazu stellt man die Vorstufe die C-terminal mit Lysin endet her und führt anschließend erfindungsgemäß eine Kupplungsreaktion mit Argininamid durch, so dass das amidierte Hirudinderivat entsteht. Die Herstellung der Vorstufen kann wie in der Anmeldung beschrieben über Hefesekretion erfolgen. Dazu verlängert man beispielhaft die in EP-A 0 324 712 beschriebene Gensequenz für Leu- Hirudin um ein Codon für Lysin und verfährt wie in der Patentschrift beispielhaft beschrieben zur Herstellung des um Lysin verlängerten Hirudins weiter. Alternativ kann man auch den Weg der Sekretion der Vorstufe durch Bakterien verfolgen. Dazu kann man z.B. die in der Patentanmeldung EP-A 1 216 259 beschriebene Technologie nutzen.

Die nachfolgenden Beispiele dienen der Illustrierung der vorliegenden Erfindung und sind in keiner Weise in Bezug auf die Gegenständer der vorliegenden Erfindung limitierend auszulegen.

### Beispiel 1: Synthese einer E. coli spezifischen DNS- Sequenz kodierend für AVE (1-43)

Zunächst wurde die Gensequenz SEQ ID No. 6 kodierend für das Peptid AVE (1-43) (SEQ ID No. 7) hergestellt:

### SEQ ID No. 6:

### SEQ ID No. 7:

HGEGTFTSDL SKQMEEEAVR LFIEWLKNGG PSSGAPPSKK KKK

Die Synthese der Gensequenz erfolgte mittels PCR-Technologie. Dazu wurden die folgenden 5 Primer mittels chemischer DNS-Synthese hergestellt. Diese Synthese erfolgte mittels des Expedite^{Tm} DNS-Synthese-Systems (Fa. Applied Biosystems).
a) Primer zp5u hat die Sequenz (SEQ ID No. 8):
   5'- TTTTTTAAGCTTGCACGGTGAAG-3'
   SEQ ID No. 8 umfasst den Bereich 1-23 des Sinn-Stranges (,sense'). Das Triplett CAC kodiert Histidin als erste Aminosäure des Zielpeptides.
b) Primer zp3a hat die Sequenz (SEQ ID No. 9): SEQ ID No. 9 umfasst den Bereich 1-59 des Gegenstranges (,antisense').
c) Primer zp3b hat die Sequenz (SEQ ID No. 10): SEQ ID No. 10 umfasst den Bereich 40-108 des Gegenstranges (,antisense').
d) Primer zp3c hat die Sequenz (SEQ ID No. 11): SEQ ID No. 11 umfasst den Bereich 91-159 des Gegenstranges (,antisense'). Das antisense-Triplett CTT kodiert die letzte Aminosäure (AS₄₃) des Zielpeptides.
e) Primer zp3d hat die Sequenz (SEQ ID No. 12): SEQ ID No. 12 umfasst den Restbereich des Gegenstranges (,antisense').

Mit den Primern wurden in Folge 4 PCR-Reaktionen unter Standard-Bedingungen bei 54°C durchgeführt. In Reaktion 1 wurden je 100ng der Primer zp3a und zp5u eingesetzt. Die PCR -Zyklenzahl war 5. In der zweiten Reaktion wurde 1/40 der Reaktion mit je 100ng der Primer zp5u und zp3b in 10 Zyklen umgesetzt. In Reaktion 3 wurden in weiteren 10 Zyklen 1/40 des Produktes der Reaktion 2 mit je 100ng der Primer zp5u und zp3c umgesetzt. Schließlich wurde in 25 PCR Zyklen mit 1/40 der Ausbeute aus Reaktion 3 und den Primern zp5u und zp3d das gewünschte DNS-Fragment synthetisiert, dessen Länge gelelektrophoretisch kontrolliert wurde. Das gewünschte DNS-Fragment wurde gereinigt und mit den Restriktionsenzymen EcoR1 und anschließend mit Hind3 nach Angaben des Herstellers (New England Biolabs) umgesetzt.

Parallel wurde DNS des Plasmides pUC19 (New England Biolabs) mit den Enzymen EcoR1 und Hind3 umgesetzt. Die Fragmente der Spaltansätze wurden über ein 1,2%-iges Agarosegel separiert und anschließend das Restvektorfragment aus pUC19 und das gewünschte Produkt aus Reaktion 4 isoliert. Die gereinigten Fragmente wurden in einer T4-Ligase-Reaktion miteinander über Nacht bei 16°C ligiert. Anschließend wurden kompetente E.coli Zellen (Stratagene, Stamm E. coli XL10 Gold), mit dem Ligationsansatz transformiert und auf Agarplatten enthaltend 25mg/l Ampicillin ausplattiert. Aus den Einzelklonen wurde Plasmid-DNS isoliert und mittels DNS-Sequenzanalyse charakterisiert.

Die Plasmid-DNS des gewünschten Fragmentes erhielt die Bezeichnung pSCHPUCZP1-43 und diente als Ausgangsmaterial zur Herstellung von Expressionsvektoren zur Synthese der Verbindungen der Formel I in E.coli K12 Zellen.

### Beispiel 2: Konstruktion eines Expressionsvektors für AVE (1-43)

US5496924, deren Inhalt hiermit ausdrücklich durch Referenz in die vorliegende Anmeldung aufgenommen wird, schlägt ein Expressionssystem vor, das grundsätzlich die Herstellung von maßgeschneiderten Fusionsproteinen erlaubt. Der Vorteil des Systems liegt darin, dass man Fusionsproteine mit kleinem Ballastanteil herstellen kann. Das Expressionsystem wird beispielhaft in der Anmeldung verwendet. Fusioniert man die Sequenzabschnitte A-B über die Enterokinase-Erkennungssequenz DDDDK mit AVE (1-43), so erhält man ein Fusionsprotein, mit folgender Gen- und Aminosäuresequenz (SEQ ID No. 13 und No. 14):

### SEQ ID No. 13:

Die Codone ATG und AAG markieren die erste und die letzte Aminosäure des Fusionspeptides.

### SEQ ID No. 14:

MAPTSSSTKK TQLQLEHLLL DLQMILNGIN NYKNPKLTRI DDDDKHGEGT FTSDLSKQME EEAVRLFIEW LKNGGPSSGA PPSKKKKK

Die Herstellung der kodierenden Gensequenz erfolgte mittels PCR-Technologie. Dazu wurden folgende Primer synthetisiert:

### 1) Primer psw3_zpcolf (SEQ ID No. 15):

5'-CGTATCGACGATGACGATAAACACGGTGAAGGTACCTTC-3'

Die Sequenz des Primers überdeckt dabei die Enterokinaseerkennungstelle und den Anfang der AVE ₁₋₄₃ kodierenden Sequenz.

### 2) Primer psw3_zpcolrev (SEQ ID No. 16):

5'-GTGTTTATCGTCATCGTCGATACGCGTCAGTTTCGG-3'

Die Sequenz entspricht dabei einer synthetischen von Interleukin2 abgeleiteten Sequenz, die entsprechend Tabelle I von US5496924 die Aminosäuren 34 -38 sowie 2/3 des Codons für die Aminosäure Methionin überdeckt. Der Rest der Primersequenz überlappt mit Primer psw3_zpcolf.

### 3) pBprimef1 (SEQ ID No. 17):

5'-TGAGCGGATAACAATTTCACAC-3'

Der Primer hybridisiert stromaufwärts mit der EcoRI-Schnittstelle, die in Plasmid pK50 (vgl. Fig. 33 der US5496924) enthalten ist.

### 4) psw3_ave_1-43_rev mit Hind3 Schnittstelle (SEQ ID No. 18):

5'-TTTTTTAAGCTTGCGGCCGCACGTGCATGCTATTATCACTT

Zwei PCR's wurden parallel durchgeführt. Die eine wurde auf DNS des Plasmides pK50 mit dem Primerpaar pBprimef1 und psw3_zpcolrev bei 50oC und die andere Reaktion mit dem Primerpaar psw3_zpcolf und psw3_ave_1-43_rev bei 54oC auf DNS des Plasmides pSCHPUCZP1-43 durchgeführt. Die PCR Ausbeuten wurden nach gelelektrophoretischer Auftrennung gereinigt, je ein Aliquot im Verhältnis 1:1 gemischt und anschließend in einer dritten PCR mit dem Primerpaar pBprimef1 und psw3_ave_1-43_rev umgesetzt. Die PCR Ausbeute wurde mit den Enzymen EcoR1 und Hind3 umgesetzt und in das parallel mit diesen Enzymen geöffnete Plasmid pK50 in einer T4-Ligasereaktion eingesetzt. Kompetente E.coli BL21 Zellen werden mit dem Ligationsgemisch transformiert und auf selektivem Agar, der 25mg/l Ampicillin enthielt, ausplattiert. Von einigen Klonen wurde Plasmid DNS re-isoliert und mittels PCR und anschließender DNS-Sequenzanalyse analysiert. Richtige Plasmide erhalten den Namen pBZP43. E. coli BL21:pBZP43 Klone werden auf Expression des Fusionsproteins überprüft. Dies geschieht in analoger Weise zu Beispiel 14 des Patents US5496924. Die Expressionsprodukte wurden massenspektrometrisch und über SDS-PAGE analysiert und der N-Terminus mittels Proteinsequenzanalyse bestimmt. Ein geeigneter Klon zur Fermentierung größerer Materialmengen wurde ausgewählt.

### Beispiel 3: Konstruktion eines Expressionsvektors für AVE (1-39)

Plasmid pBZP43 dient als Template für die PCR-Reaktion, die mit den Primern pBprimef1 (Bsp.2) und psw3_ave_39rev durchgeführt wird. Das PCR Produkt wird mit den Restiktionsenzyme EcoRI und NotI entsprechend den Angaben der Enzymhersteller umgesetzt und in einer T4 - Ligase Reaktion in das EcorI/NotI geöffnete Plasmid pBZP43 insertiert. Es entseht das Plasmid pBZP39, mit dem wie in Beispiel 2 beschrieben weiter fortgefahren wird.
psw3_ave_39rev (SEQ ID No. 19):
5'-TTTTTTGCGGCCGCACGTGCATGCTATTATCATTTCGAAGGCGGAGCACC-3'

Das Triplett TTT kodiert Lysin in Position 39.

### Beispiel 4: Konstruktion eines Expressionsvektors für AVE (1-38-Arg)

Plasmid pBZP43 dient als Template für die PCR-Reaktion, die mit den Primern pBprimef1 (Bsp.2) und psw3_ave38_argrev durchgeführt wird. Das PCR Produkt wird mit den Restiktionsenzyme EcoRI und NotI entsprechend den Angaben der Enzymhersteller umgesetzt und in einer T4-Ligase Reaktion in das EcoRI/NotI geöffnete Plasmid pBZP43 insertiert. Es entseht das Plasmid pBZP38arg, mit dem wie in Besipiel 2 beschrieben weiter fortgefahren wird.
Primer: psw3_ave38_argrev (SEQ ID No. 20):

Das Triplett AGG kodiert Arginin in Position 39.

### Beispiel 5: Fermentation der in Beispiel 2-4 konstruierten Stämme

Die Fermentation erfolgte mit geringen Abweichungen wie in der Deutschen Patentanmeldung DE10 2004 058306.4 Bsp. 3 beschrieben. E. coli BL21-Zellen, transformiert mit verschiedenen, für Zielpeptid-Derivate(-Fusionsprotein) kodierenden Plasmid-Vektoren, wurden in Mineralsalzmedium oder Komplexmedium (siehe Beispiel 1) bei 30°C oder 37°C und einem pH-Wert von 7,0 in einem Fermenter kultiviert. Die pH-Einstellung erfolgte mit einer NH₄⁺-Lösung (26% in Wasser). Die Belüftung der Kultur wurde durch eine Regelungsstrategie, die den Gelöstsauerstoff in der Kulturbrühe konstant bei 30% hielt, sichergestellt. Für Fed Batch Prozesse in Mineralsalzmedium wurde nach Beendigung der Batch-Phase eine Glucoselösung (60% w/v) zugefüttert (8 g/L/h bis 26g/L/h). Die Induktion der Proteinexpression erfolgte durch Zugabe von IPTG (1-4 mM Endkonz. (f.c.)). Die Zeitdauer der Induktion betrug 6-8 h. Die Expression der Zielproteine wurde durch SDS-Polyacrylamid-Gelelektrophorese (SDS-PAGE) nachgewiesen.

Die Expression von AVE-Vorstufenfusionsprotein in E. coli BL21/pBZP43 wurde wie nachfolgend beschrieben durchgeführt:
Aus einer bei -80°C gelagerten Dauerkultur von E. coli BL21-Zellen wurden 100 µL Zellsuspension entnommen und in 0,5 L Vorkulturmedium bei 37°C schüttelnd für 10-16 h inkubiert. Die Hauptkultur im Fermenter wurde auf eine Animpfdichte von 0,01 bis 0,05 OD₆₀₀ mit der entsprechenden Menge Vorkultur inokuliert.

Vorkulturmedium:
5 g/L Bacto trypton
10 g/L Hefeextrakt
5 g/L NaCl

Hauptkulturmedium:

Definiertes Mineralsalzmedium (Minimalmedium) basierend auf Glukose als Kohlenstoffquelle (Jeffrey H. Miller: Experiments in Molecular Genetics, Cold Spring Harbor Laboratory (1972)).

Nach Aufbrauchen der initial im Hauptkulturmedium vorhandenen Glucose wurde eine Glucoselösung zugefüttert. Die Proteinexpression durch Zugabe von IPTG (1 mM f.c.) induziert und die maximale Expression des Fusionsproteins nach der Induktion beobachtet. Unter Verwendung z.B. des SDS-PAGE Analysensystems der Firma Novex (NuPage^{®} Novex 12% Gelsystem, Invitrogen^{™}) wurden entsprechend der Angaben des Herstellers in der Fermentation je 0,02 OD₆₀₀ₙₘ Zellsuspension, die zu unterschiedlichen Kultivierungszeitpunkten dem Fermenter entnommen wurden, analysiert.

### Beispiel 6 : Reinigung der in Beispiel 5 hergestellten Fusionsproteine

1000 g Biomasse eines rekombinanten E. coli-Stammes wurden in 1000 ml Tris-Puffer (50 mM Tris/HCl, pH 7,4) resuspendiert. Der Zellaufschluss erfolgte durch zweimalige Hochdruckhomogenisation (Rannie-Hochdruckhomogenisator, 1000 bar). Durch Zugabe von Benzonase (1000 U/L) und Magnesiumchlorid (10 mM) erfolgte der Verdau der genomischen DNS für 1,5 Stunden. Die Aufreinigung des Fusionsproteins wurde durch Expanded-Bed-Chromatographie durchgeführt. Hierzu wurde das Zellhomogenat auf 10 Liter mit Puffer (50 mM TrislHcl, pH 7,4) verdünnt und direkt auf eine zuvor mit Puffer (50 mM Tris/HCl pH 7,4) äquilibrierte Chromatographiesäule (Streamline SP XL, GE Healthcare) aufgetragen. Nach dem Probenauftrag erfolgte ein Waschschritt mit Äquilibrierungspuffer (6 Säulenvolumen), gefolgt von einem weiteren Waschschritt mit 7% Hochsalzpuffer (50 mM Tris/HCl pH 7,4; 1M NaCl). Die Elution erfolgte durch Spülen mit 10 Säulenvolumen 20% Hochsalzpuffer. Die Überprüfung des Elutionspools erfolgte über SDS-Gelelektrophorese (NuPage^{®} Novex 12% Gelsystem, Invitrogen) und HPLC. Der Fusionsprotein-Pool wurde nach Diafiltration in Enterokinasepuffer (50 mM Tris/HCl pH 7,4; 50 mM NaCl, 2mM CaCl₂) zur Proteasespaltungsreaktion eingesetzt. Die Spaltung der Fusionsproteine erfolgte mittels Enterokinase (Invitrogen) in Enterokinase-Puffer (20 mM Tris/HCl, 50 mM NaCl, 2 mM CaCl₂ pH 7,4) nach Angaben des Herstellers.

### Beispiel 7: Trennung der Spaltprodukte aus der Enterokinase-Spaltreaktion

Die Trennung der Spaltprodukte erfolgt entsprechend Beispiel 6 der Deutschen Patentanmeldung DE102004058306.4. Nach der Spaltung der Fusionsproteine mittels Enterokinase wurden die Spaltprodukte voneinander durch Ionenaustauschchromatographie (Source 30S, Amersham Biosciences) getrennt. Die Ionenstärke der Lösung wurde auf ca. 7 mS/cm durch Verdünnen mit H₂O gebracht. Nach Aufgabe der Proteinlösung auf die zuvor äquilibrierte Säule (20 mM Tris/HCl, pH 7.4; mit NaCl auf eine Leitfähigkeit von ca. 7 mS/cm eingestellt) wurde nicht gebundenes Material mit 15% Puffer B (20 mM Tris/HCl, pH 7.4; 500 mM NaCl) herausgewaschen. Die Elution der AVE Peptidvorstufen erfolgte durch Anlegen eines Gradienten über 10 Säulenvolumen auf 100% Puffer B. Die Identifizierung AVE-Vorläufer-haltiger Fraktionen erfolgte durch SDS-Gelelektrophorese, HPLC und massenspektrometrisch. Die entsprechenden Fraktionen wurden vereinigt, entsalzt und nach Entfernen von organischem Lösungsmittel lyophilisiert.

### Beispiel 8: Peptidkupplung von AVE (1-39) mit H-Lys(Boc)-NH₂

0,2 mg AVE (1-39) (MW 4218; 0.047 µmol; 1 g/L Endkonzentration) werden in ein 1,5 mL Polypropylen-Reaktionsgefäß gewogen. 11 µL 0,1 M Pyridin-Acetatpuffer pH 5,6, 60 µL einer 129 g/L Lösung von H-Lys(Boc)-NH₂.HCl in 0,1 M Pyridin-Acetatpuffer pH 5,6 (enthält 7,75 mg H-Lys(Boc)-NH₂.HCl = 27,5 µmol = 585 mol H-Lys(Boc)-NH₂.HCl pro mol AVE (1-39)) und 119 µL DMF werden hinzugefügt. Die klare Lösung wird auf 12 °C temperiert. Die Reaktion wird durch die Zugabe von 10 µL einer 2 g/L Trypsinlösung in Wasser (enthält 0,02 mg Trypsin = 0,002 g Trypsin pro g AVE (1-39)) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9- vol einer Lösung aus 17 % Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 9 : Peptidkupplung von AVE (1-43) mit H-Lys-NH₂

3,85 mg AVE (1-43) (MW 4731; 0.814 µmol; 20 g/L Endkonzentration) werden in ein 1,5 mL Polypropylen-Reaktionsgefäß gewogen. 41 µL einer 620 g/L Lösung von H-Lys-NH₂.2HCl in 0,1 M Natrium-Acetatpuffer pH 5,8 (enthält 25,6 mg H-Lys-NH₂.2HCl = 117.5 µmol = 144 mol H-Lys-NH₂.2HCl pro mol AVE (1-43)), 116 µL DMF und 32 µL 0,1 M Natrium-Acetatpuffer pH 5,8 werden hinzugefügt. Die klare Lösung wird auf 12 °C temperiert. Die Reaktion wird durch die Zugabe von 2,9 µL einer 20 g/L Trypsinlösung in Wasser (enthält 0,06 mg Trypsin = 0,015 g Trypsin pro g AVE (1-43)) gestartet. Die Reaktionslösung wird bei 12 °C unter Schütteln bei 900 min⁻¹ inkubiert. Proben zur Prozesskontrolle werden regelmäßig entnommen und durch Verdünnung mit 9 vol einer Lösung aus 17 % Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 10: Peptidkupplung von AVE (1-39) mit H-Lys-NH₂

0,2 mg AVE (1-39) (MW 4218; 0.047 µmol; 1 g/L Endkonzentration) werden in ein 1,5 mL Polypropylen-Reaktionsgefäß gewogen. 11 µL 0,1 M Pyridin-Acetatpuffer pH 5,6, 60 µL einer 100 g/L Lösung von H-Lys-NH₂.2HCl in 0,1 M Pyridin-Acetatpuffer pH 5,6 (enthält 6,0 mg H-Lys-NH₂.2HCl = 27,5 µmol = 585 mol H-Lys-NH₂.2HCl pro mol AVE (1-39)) und 119 µL DMF werden hinzugefügt. Die klare Lösung wird auf 12 °C temperiert. Die Reaktion wird durch die Zugabe von 10 µL einer 2 g/L Trypsinlösung in Wasser (enthält 0,02 mg Trypsin = 0,002 g Trypsin pro g AVE (1-39)) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9- vol einer Lösung aus 17% Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 11: Peptidkupplung von AVE (1-39) mit H-Arg-NH₂

0,2 mg AVE (1-39) (MW 4218; 0.047 µmol; 1 g/L Endkonzentration) werden in ein 1,5 mL Polypropylen-Reaktionsgefäß gewogen. 11 µL 0,1 M Pyridin-Acetatpuffer pH 5,6, 60 µL einer 113 g/L Lösung von H-Arg-NH₂.2HCl in 0,1 M Pyridin-Acetatpuffer pH 5,6 (enthält 6,77 mg H-Arg-NH₂.2HCl = 27,5 µmol = 585 mol H-Arg-NH₂.2HCl pro mol AVE (1-39)) und 119 µL DMF werden hinzugefügt. Die klare Lösung wird auf 12 °C temperiert. Die Reaktion wird durch die Zugabe von 10 µL einer 2 g/L Trypsinlösung in Wasser (enthält 0,02 mg Trypsin = 0,002 g Trypsin pro g AVE (1-39)) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9-vol einer Lösung aus 17 % Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 12: Peptidkupplung von AVE (1-43) mit H-Arg-NH₂

0,25 mg AVE (1-43) (MW 4731; 0.047 µmol; 1,1 g/L Endkonzentration) werden in ein 1,5 mL Polypropylen-Reaktionsgefäß gewogen. 11 µL 0,1 M Pyridin-Acetatpuffer pH 5,6, 60 µL einer 113 g/L Lösung von H-Arg-NH₂.2HCl in 0,1 M Pyridin-Acetatpuffer pH 5,6 (enthält 6,77 mg H-Arg-NH₂.2HCl = 27,5 µmol = 585 mol H-Arg-NH₂.2HCl pro mol AVE (1-43)) und 119 µL DMF werden hinzugefügt. Die klare Lösung wird auf 12 °C temperiert. Die Reaktion wird durch die Zugabe von 10 µL einer 2 g/L Trypsinlösung in Wasser (enthält 0,02 mg Trypsin = 0,002 g Trypsin pro g AVE (1-43)) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9- vol einer Lösung aus 17 % Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 13: Peptidkupplung von AVE (1-38)-Arg mit H-Lys-NH₂

0,2 mg AVE (1-38)-Arg (MW 4246; 0.047 µmol; 1 g/L Endkonzentration) werden in ein 1,5 mL Polypropylen-Reaktionsgefäß gewogen. 11 µL 0,1 M Pyridin-Acetatpuffer pH 5,6, 60 µL einer 100 g/L Lösung von H-Lys-NH₂.2HCl in 0,1 M Pyridin-Acetatpuffer pH 5,6 (enthält 6,0 mg H-Lys-NH₂.2HCl = 27,5 µmol = 585 mol H-Lys-NH₂.2HCl pro mol AVE (1-38)-Arg) und 119 µL DMF werden hinzugefügt. Die klare Lösung wird auf 12 °C temperiert. Die Reaktion wird durch die Zugabe von 10 µL einer 2 g/L Trypsinlösung in Wasser (enthält 0,02 mg Trypsin = 0,002 g Trypsin pro g AVE (1-38)-Arg) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9- vol einer Lösung aus 17% Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure auf einen pH-Wert unter 2,5 angesäuert und chromatographisch gereinigt.

### Beispiel 14: Peptidkupplung von AVE (1-43) mit H-Lys(Boc)-NH₂

20 mg AVE (1-43) (MW 4731; 1,058 µmol; 20 g/L Endkonzentration) werden in ein 2 mL Polypropylen-Reaktionsgefäß gewogen. 370 µL einer 482 g/L Lösung von H-Lys(Boc)-NH₂.HCl in 0,1 M Natrium-citratpuffer pH 5,5 (enthält 155 mg H-Lys(Boc)-NH₂.HCl = 550 µmol = 130 mol H-Lys(Boc)-NH₂.HCl pro mol AVE (1-43)) und 600 µL DMF werden hinzugefügt. Die klare Lösung wird auf 12 °C temperiert. Die Reaktion wird durch die Zugabe von 30 µL einer 3,3 g/L Trypsinlösung in Wasser (enthält 0,1 mg Trypsin = 5 mg Trypsin pro g AVE (1-43)) gestartet. Die Reaktionslösung wird bei 12 °C unter Schütteln bei 1000 min⁻¹ inkubiert. Proben zur Prozesskontrolle werden regelmäßig entnommen und durch Verdünnung mit 9-vol einer Lösung aus 17 % Wasser, 17 % Acetonitril und 64 % Trifluoressigsäure abgestoppt. Der Reaktionsverlauf wird durch LC-MS verfolgt. Nach Erreichen des Ausbeutemaximums wird die Reaktion durch Zugabe von Trifluoressigsäure bis zu einer Endkonzentration von 50 % (v/v) angesäuert. Hierdurch wird die Reaktion gestoppt und gleichzeitig die Boc-Schutzgruppe quantitativ entfernt. Das Reaktionsprodukt wird chromatographisch gereinigt.

### Beispiel 15: Reinigung der amidierten AVE-Derivate

Das Reaktionsgemisch der Kupplungsreaktionen wird anschließend über RP-Chromatographie mit Amberchrom CG300 XT 20 als Trägermaterial getrennt und das amidierte Zielpeptid aus der entsprechenden Eluat-Fraktion anschließend über einen lonentauscherschritt mit Source 30S als Träger isoliert werden. Nach Entsalzung über Amberchromsäulen liegt das Produkt zur Formulierung als Arzneimittel vor. Die Identität der Struktur des Produktes wurde mittels MALDI-MS- und NMR-Analytik nachgewiesen.

### Beispiel 16: Herstellung von Leu-Hirudin ₁₋₆₅ Lys - Arg-NH₂

Beispiel 1 der Patentanmeldung EP-A 1 216 259 beschreibt die Herstellung eines Expressionsplasmides, das die Sekretion von Leu-Hirudin in bakterielle Überstände erlaubt. DNA des Plasmides wird als Template in einer Standard PCR eingesetzt. Als Vorwärtsprimer verwendet man in der Reaktion die in dem Beispiel beschriebe Sequenz smompaf2. Als Gegenprimer verwendet man ein Oligonukleotid hir_lys66_rev (SEQ ID NO.: 21), das folgende Sequenz aufweist:

Dabei kodiert das unterstrichene Codon für Lysin. Von Position 22 bis Position 40 (Ende) ist die Sequenz komplementär zu dem Sequenzabschnitt 178 -195 der in Tabelle 1 der Anmeldung EP-A 1 216 259 dargestellten Sequenz.

Wie in dem Beispiel beschrieben, wird die PCR durchgeführt, das Produkt mit den Restriktionsenzymen EcoR1 und Hind3 verdaut und in den entsprechend geöffneten Vektor pJF118 insertiert. Nach Charakterisierung wird DNA wie in Beispiel 11 der Patentanmeldung EP-A 1 216 259 nach E.coli K12 transformiert und das Zwischenprodukt wird exprimiert und gereinigt. Dann erfolgt entsprechend Beispiel 13 der vorliegenden Anmeldung entsprechend den Molaritäten die Umsetzung mit Argininamid zu Leu-Hirudin ₁₋₆₅ Lys - Arg-NH₂. Führt man die Expression direkt in dem als Zwischenwirtsstamm Benutzten Stamm MC1061 durch, so findet man Produkt auch im periplasmatischen Raum. Dies macht zur Isolation des Zwischenproduktes einen Zellaufschluss entsprechend bekannter Methoden als zusäztlichen Aufarbeitungsschritt nötig.

### Beispiel 17: Analyse von Peptidkopplungsreaktionen an AVE-Derivaten

Die analytische Verfolgung der Kopplungsreaktionen nach Beispielen 8-14 erfolgt durch RP-HPLC an einer Säule Symmetry 300 150x4,6 mm, 5 µm von Waters. Als Eluenten dient 0,1 % (v/v) Ameisensäure (Eluent A) und Acetonitril mit 0,1 % Ameisensäure (Eluent B). Es wird in einem linearen Gradienten über 15 min von 20 auf 50 % B bei einer Säulentemperatur von 60 °C und einer Flussrate von 1 mL/min eluiert. Die Detektion erfolgt bei 215 nm. Es werden üblicherweise 5 µL einer 1:25 verdünnten Reaktionsprobe injiziert. Die entschützten AVE-Derivate werden mit Retentionszeiten zwischen 7 und 10 min detektiert.

## Patentansprüche

1. Verfahren zur Herstellung C-terminal amidierter di- oder polybasischer Peptide der allgemeinen Formel I
(AS)-X-NH₂ (I),
wobei
AS ein Peptid charakterisiert durch die Sequenz oder und
X Lysin oder Arginin bedeutet;
worin ein Peptid charakterisiert durch die Sequenz mit H-Arg-NH₂ oder H-Lys-NH₂ in Gegenwart eines Enzyms mit der biologischen Aktivität von Trypsin, wobei das Enzym die Fähigkeit hat, eine Peptidbindung C-terminal zu basischen Aminosäuren zu spalten, umgesetzt wird, und gegebenenfalls die erhaltene Verbindung der Formel I proteinchemisch aufgereinigt wird.

2. Verfahren gemäß Anspruch 1, wobei im Falle der Herstellung des Peptids charakterisiert durch die Sequenz SEQ ID NO. 1 das molare Verhältnis des Peptids charakterisiert durch die Sequenz ID NO. 7 und H-Lys-NH₂ 1 : 144 und im Falle der Herstellung des Peptids charakterisiert durch die Sequenz ID NO. 4 das molare Verhältnis des Peptids charakterisiert durch die Sequenz ID NO. 7 und H-Arg-NH₂ 1:585 beträgt.

3. Verfahren gemäß Anspruch 1 oder 2, worin
a) ein Fusionspeptid exprimiert wird, das ein Peptid charakterisiert durch die Sequenz ID NO. 7 enthält;
b) das Peptid charakterisiert durch die Sequenz ID NO. 7 mittels enzymatischer Spaltung aus besagtem Fusionspeptid freigesetzt wird;
c) das Zwischenprodukt aus Schritt b), gegebenenfalls nach proteinchemischer Reinigung, in Gegenwart eines Enzyms mit der biologischen Aktivität von Trypsin mit H-Lys-NH₂ oder H-Arg-NH₂ umgesetzt wird; und
d) gegebenenfalls die erhaltene Verbindung an Peptide charakterisiert durch die Sequenzen SEQ ID NO. 1 oder 4 der Formel I proteinchemisch aufgereinigt und isoliert wird.

4. Verfahren nach Anspruch 3, worin die Abspaltung von dem Fusionsprotein mittels Enterokinase, Faktor Xa, Genenase, Thrombin oder Trypsin geschieht.

5. Verfahren nach Anspruch 4, worin das Fusionsprotein exprimiert wird in einem Expressionssystem ausgewählt aus der Gruppe enthaltend E.coli , S.carnosus, Salmonella, Bacillus subtilis, Pseudomonas fluorescens, K.lactis, P.pastoris, Schizosaccharomyces pombe und S.cerevisiae.

## Claims

1. A process for preparing C-terminally amidated di- or polybasic peptides of the general formula I
(AA)-X-NH₂ (I),
where
AS is a peptide **characterized by** the sequence or and
X is lysine or arginine;
wherein a peptide **characterized by** the sequence
HGEGTFTSDL SKQMEEEAVR LFIEWLKNGG PSSGAPPSKK KKK (SEQ ID No. 7)
is reacted with H-Arg-NH₂ or H-Lys-NH₂ in the presence of an enzyme having the biological activity of trypsin in that the enzyme is capable of cleaving a peptide bond C-terminally to basic amino acids, and optionally the resulting compound of formula I is subjected to protein chemical purification.

2. The process as claimed in claim 1, wherein in the case of the production of the peptide **characterized by** the sequence SEQ ID NO. 1 the molar ratio of the peptide **characterized by** the sequence ID NO. 7 and H-Lys-NH₂ is 1:144 and in the case of the preparation of the peptide **characterized by** the sequence ID NO. 4 the molar ratio of the peptide **characterized by** the sequence ID NO. 7 and H-Arg-NH₂ is 1:585.

3. The process as claimed in claim 1 or 2, in which
a) a fusion peptide which comprises a peptide **characterized by** the sequence ID NO. 7 is expressed;
b) the peptide **characterized by** the sequence ID NO. 7 is liberated from said fusion peptide by enzymatic cleavage;
c) the intermediate from step b) is, where appropriate after protein chemical purification, reacted in the presence of an enzyme having the biological activity of trypsin with H-Lys-NH₂ or H-Arg-NH₂ ; and
d) where appropriate the resulting compound of peptides **characterized by** the sequences SEQ ID NO. 1 or 4 of the formula I is subjected to protein chemical purification and isolation.

4. The process as claimed in claim 3, in which the elimination from the fusion protein takes place by means of enterokinase, factor Xa, Genenase, thrombin or trypsin.

5. The process as claimed in claim 4, in which the fusion protein is expressed in an expression system selected from the group comprising E. coli, S. carnosus, Salmonella, Bacillus subtilis, Pseudomonas fluorescens, K. lactis, P. pastoris, Schizosaccharomyces pombe and S. cerevisiae.

## Revendications

1. Procédé de préparation de peptides di- ou polybasiques amidés C-terminaux de formule générale I
**(AS)-X-NH₂** **(I),**
dans laquelle
AS est un peptide **caractérisé par** la séquence ou et
X est la lysine ou l'arginine ;
dans lequel on fait réagir un peptide **caractérisé par** la séquence avec H-Arg-NH₂ ou H-Lys-NH₂ en présence d'une enzyme ayant l'activité biologique de la trypsine, l'enzyme ayant la capacité à dissocier une liaison peptidique en position C-terminale d'acides aminés basiques, et éventuellement on purifie par la chimie des protéines le composé obtenu de formule I.

2. Procédé selon la revendication 1, dans lequel, dans le cas de la préparation du peptide **caractérisé par** la séquence SEQ ID N° 1, le rapport en moles entre le peptide **caractérisé par** la séquence SEQ ID N° 7 et H-Lys-NH₂ est de 1:144 et, dans le cas de la préparation du peptide **caractérisé par** la séquence SEQ ID N° 4, le rapport en moles entre le peptide **caractérisé par** la séquence SEQ ID N° 7 et H-Arg-NH₂ est de 1:585.

3. Procédé selon la revendication 1 ou 2, dans lequel
a) on exprime un peptide de fusion qui contient un peptide **caractérisé par** la séquence SEQ ID N° 7 ;
b) on libère dudit peptide de fusion, par dissociation enzymatique, le peptide **caractérisé par** la séquence SEQ ID N° 7 ;
c) on fait réagir avec H-Lys-NH₂ ou H-Arg-NH₂ le produit intermédiaire de l'étape b), éventuellement après purification par la chimie des protéines, en présence d'une enzyme ayant l'activité biologique de la trypsine ; et
d) éventuellement, on purifie par la chimie des protéines et on isole de la formule I le composé obtenu de peptides **caractérisés par** les séquences SEQ ID N° 1 ou 4.

4. Procédé selon la revendication 3, dans lequel la dissociation par rapport à la protéine de fusion a lieu à l'aide d'entérokinase, de facteur Xa, de génénase, de thrombine ou de trypsine.

5. Procédé selon la revendication 4, dans lequel la protéine de fusion est exprimée dans un système d'expression choisi dans le groupe contenant E. coli, S. carnosus, Salmonella, Bacillus subtilis, Pseudomonas fluorescens, K. lactis, P. pastoris, Schizosaccharomyces pombe et S. cerevisiae.


### SEQUENZPROTOKOLL

<110> Sanofi-Aventis Deutschland GmbH
<120> verfahren zur Amidierung von Polypeptiden mit C-terminalen basischen Aminosäuren unter Verwendung spezifischer Endoproteasen
<130> DE 2005/042
<140>
   <141>
<160> 21
<170> PatentIn Ver. 2.1
<210> 1
   <211> 44
   <212> PRT
   <213> Künstliche Sequenz
<220>
<223> Beschreibung der künstlichen Sequenz: Exendin-4-Derivat
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 40
   <212> PRT
   <213> Künstliche Sequenz
<220>
<223> Beschreibung der künstlichen Sequenz: Exendin-4-Derivat
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys
           35                  40
<210> 3
   <211> 40
   <212> PRT
   <213> Künstliche Sequenz
<220>
<223> Beschreibung der künstlichen sequenz: Exendin-4-Derivat
<400> 3
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Arg
           35                  40
<210> 4
   <211> 44
   <212> PRT
   <213> Künstliche sequenz
<220>
<223> Beschreibung der künstlichen Sequenz: Exendin-4-Derivat
<400> 4
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Arg
           35                  40
<210> 5
   <211> 40
   <212> PRT
   <213> Künstliche Sequenz
<220>
<223> Beschreibung der künstlichen sequenz: Exendin-4-Derivat
<400> 5
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Arg Lys
           35                  40
<210> 6
   <211> 192
   <212> DNA
   <213> Künstliche sequenz
<220>
<223> Beschreibung der künstlichen sequenz: Gen für Sequenz ID Nr. 7
<400> 6
   ttttttaagc ttgcacggtg aaggtacctt cacctccgac ctgtccaaac agatggaaga 60
   agaagctgtt cgtctgttca tcgaatggct gaaaaacggt ggtccgtcct ccggtgctcc 120
   gccttcgaaa aagaagaaaa agtgataata gcatgcacgt gcggccgcac ctggtcgacg 180
   aattcaaaaa aa                                                    192
<210> 7
   <211> 44
   <212> PRT
   <213> Künstliche Sequenz
<220>
<223> Beschreibung der künstlichen Sequenz:
Exendin-4-Derivat
<400> 7
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Ser Lys Lys Lys Lys Lys Lys
   35 40
<210> 8
   <211> 23
   <212> DNA
   <213> Künstliche Sequenz
<220>
   <223> Beschreibung der künstlichen Sequenz: Primer
<400> 8
   ttttttaagc ttgcacggtg aag 23
<210> 9
   <211> 59
   <212> DNA
   <213> Künstliche sequenz
<220>
<223> Beschreibung der künstlichen Sequenz: Primer
<400> 9
   cttccatctg tttggacagg tcggaggtga aggtaccttc accgtgcaag cttaaaaaa 59
<210> 10
   <211> 69
   <212> DNA
   <213> Künstliche sequenz
<220>
   <223> Beschreibung der künstlichen sequenz: Primer
<400> 10
   ggacggacca ccgtttttca gccattcgat gaacagacga acagcttctt cttccatctg 60
   tttggacag 69
<210> 11
   <211> 69
   <212> DNA
   <213> Künstliche Sequenz
<220>
   <223> Beschreibung der künstlichen sequenz: Primer
<400> 11
   cgtgcatgct attatcactt tttcttcttt ttcgaaggcg gagcaccgga ggacggacca 60
   ccgtttttc 69
<210> 12
   <211> 52
   <212> DNA
   <213> Künstliche Sequenz
<220>
<223> Beschreibung der künstlichen sequenz: Primer
<400> 12
   ttttttgaat tcgtcgacca ggtgcggccg cacgtgcatg ctattatcac tt 52
<210> 13
   <211> 314
   <212> DNA
   <213> Künstliche Sequenz
<220>
<223> Beschreibung der künstlichen Sequenz: Gen für Sequenz ID Nr.14 <400> 13
<210> 14
   <211> 88
   <212> PRT
   <213> Künstliche Sequenz
<220>
<223> Beschreibung der künstlichen Sequenz: Fusionsprotein
<400> 14
   Met Ala Pro Thr Ser Ser Ser Thr Lys Lys Thr Gln Leu Gln Leu Glu
   1               5                   10                  15
   His Leu Leu Leu Asp Leu Gln Met Ile Leu Asn Gly Ile Asn Asn Tyr
               20                  25                  30
   Lys Asn Pro Lys Leu Thr Arg Ile Asp Asp Asp Asp Lys His Gly Glu
           35                  40                  45
   Gly Thr Phe Thr Ser Asp Leu Ser Lys Gln Met Glu Glu Glu Ala Val
       50                  55                  60
   Arg Leu Phe Ile Glu Trp Leu Lys Asn Gly Gly Pro Ser Ser Gly Ala
   65                  70                  75                  80
   Pro Pro Ser Lys Lys Lys Lys Lys
                   85
<210> 15
   <211> 39
   <212> DNA
   <213> Künstliche sequenz
<220>
<223> Beschreibung der künstlichen Sequenz: Primer
<400> 15
   cgtatcgacg atgacgataa acacggtgaa ggtaccttc 39
<210> 16
   <211> 36
   <212> DNA
   <213> Künstliche Sequenz
<220>
   <223> Beschreibung der künstlichen Sequenz: Primer
<400> 16
   gtgtttatcg tcatcgtcga tacgcgtcag tttcgg 36
<210> 17
   <211> 22
   <212> DNA
   <213> Künstliche Sequenz
<220>
   <223> Beschreibung der künstlichen Sequenz: Primer
<400> 17
   tgagcggata acaatttcac ac 22
<210> 18
   <211> 41
   <212> DNA
   <213> Künstliche Sequenz
<220>
<223> Beschreibung der künstlichen Sequenz: Primer
<400> 18
   ttttttaagc ttgcggccgc acgtgcatgc tattatcact t 41
<210> 19
   <211> 50
   <212> DNA
   <213> Künstliche Sequenz
<220>
   <223> Beschreibung der künstlichen Sequenz: Primer
<400> 19
   ttttttgcgg ccgcacgtgc atgctattat catttcgaag gcggagcacc 50
<210> 20
   <211> 52
   <212> DNA
   <213> Künstliche Sequenz
<220>
   <223> Beschreibung der künstlichen Sequenz: Primer
<400> 20
   ttttttgcgg ccgcacgtgc atgctattat catacgcgaa ggcggagcac cg 52
<210> 21
   <211> 40
   <212> DNA
   <213> Künstliche Sequenz
<220>
   <223> Beschreibung der künstlichen sequenz: Primer
<400> 21
   ttttttaagc ttctattatt tctgaaggta ttcctcaggg 40